# DEMANDE DE BREVET EUROPEEN

(11) **EP 2 532 619 A1**
(43) Date de publication de la demande: **12.12.2012**
(21) Numéro de dépôt: 11169070.7
(22) Date de dépôt: 08.06.2011
(51) Int. Cl.: B81C 1/00

(54) **Soudure anodique pour dispositif de type MEMS**

(71) Demandeur: Debiotech S.A., 1004 Lausanne (CH)
(72) Inventeur: Bianchi, François, 1920 Martigny (CH)
(74) Mandataire: Grosfillier, Philippe

(57) **Abrégé**

Dispositif comprenant une plaque comprenant une surface de silicium et une plaque comprenant une surface de verre fixées l'une à l'autre, la zone de fixation ainsi formée entre les plaques définissant une structure multicouche comprenant une première couche de protection contre une altération physique du matériau couvrant la surface de silicium et une deuxième couche de protection contre une altération physique du matériau couvrant la surface de verre; ladite structure multicouche comprenant en outre au minimum une couche additionnelle permettant de réaliser une soudure anodique entre les deux couches de protection.

## Description

### Domaine de l'invention

La présente invention se situe dans le domaine des microsystèmes électromécaniques communément appelés dispositifs de type MEMS (Micro Electro Mechanical Systems). Elle concerne plus précisément la soudure anodique entre une plaque dont une des faces est en silicium et une plaque dont une des faces est en verre. Ces deux éléments constituent les éléments de base de la plupart des dispositifs de type MEMS, microsystème comprenant un ou plusieurs éléments mécaniques, pouvant utiliser si nécessaire l'électricité comme source d'énergie, en vue de réaliser une fonction de capteur et/ou d'actionneur avec au moins une structure présentant des dimensions micrométriques, la fonction du système étant en partie assurée par la forme de cette structure

### Abréviations utilisées dans le présent texte

- ALD: Atomic Layer Deposition
- CVD: Chemical Vapor Deposition
- LPCVD: Low Pressure Chemical Vapor Deposition
- MEMS: Micro Electro Mechanical Systems
- MIP: Micro Implantable Pump
- PECVD: Plasma Enhanced Chemical Vapor Deposition
- PVD: Physical Vapor Deposition
- a-Si: Silicium amorphe

### Etat de la technique

Un système microfluidique tel qu'une pompe ou un régulateur de débit doit être protégé contre les attaques chimiques, en particulier s'il est destiné à être implanté pendant plusieurs années dans un patient, par exemple comme système de libération d'un principe actif.

En général les éléments sensibles à de telles attaques chimiques, comme les plaques en silicium ou en verre, sont recouverts d'une couche de protection. L'assemblage de ces éléments entre eux n'est donc pas toujours aisé. Un tel procédé d'assemblage a fait l'objet d'une demande de brevet portant sur un système micro-fluidique implantable [R11].

Depuis de nombreuses années, plusieurs groupes de recherche ont traité (avec succès) [R1, R4] de la possibilité de souder sur une surface de verre (Pyrex 7740 en général) une surface de silicium recouverte de nitrure de silicium.

Par ailleurs, d'autres groupes de recherche ont démontré qu'il était possible de souder par soudure anodique deux plaques de même nature (en silicium [R6, R7] ou en verre [R2, R5]) en utilisant des couches intermédiaires. Mais comme indiqué dans l'article de Knowles [R3], l'objectif dans ces études étaient de rendre possible la soudure entre deux substrats qu'il était impossible ou difficile de souder a priori, sans recours à une ou des couches intermédiaires.

Une autre approche employée utilise une technique de soudure directe (aucune utilisation de tension électrique mais utilisation de pression et de préparation de surfaces) pour souder une plaque de silicium recouverte de nitrure de silicium contre une plaque en verre [R8].

### Description générale de l'invention

L'invention décrite dans ce brevet consiste en un dispositif type MEMS comprenant une plaque avec une surface de silicium et une plaque avec une surface de verre fixées l'une à l'autre, la zone de fixation ainsi formée entre les plaques définissant une structure multicouche comprenant une première couche de protection contre une altération physique du matériau couvrant la plaque de silicium et une deuxième couche de protection contre une altération physique du matériau couvrant la surface de verre; ladite structure multicouche comprenant en outre au minimum une couche additionnelle permettant de réaliser une soudure anodique entre les deux couches de protection.

Contrairement à l'enseignement de l'état de la technique [R11] qui se limite à présenter le design d'une résistance fluidique pour une pompe implantable ayant la forme d'un réseau capillaire possédant une couche de protection pH meilleure que le verre ou le silicium, la problématique adressée par notre invention concerne le packaging d'un MEMS par soudure anodique classique d'une plaque dont une des surfaces est en silicium avec une plaque dont une des surfaces est en verre - un matériau ou un alliage dur le plus souvent constitué d'oxyde de silicium (silice SiO2, constituant principal du sable) et de fondants, tout en les protégeant l'une et l'autre contre des attaques chimiques. On utilisera plus volontiers du borosilicate comme par exemple le Pyrex 7740 ou un matériau équivalent comme par exemple ceux décrits dans la Table 1, un objectif potentiel étant de profiter de sa transparence.

Pour réaliser cette protection contre les attaques chimiques, le silicium peut être protégé par une fine couche de type Si₃N₄ ou TiN (déjà décrite dans la littérature), ou de manière plus complexe par une combinaison de deux couches : TiO₂+Si₃N₄, ou encore TiO₂+a-Si. Dans ce cas la couche de a-Si ou de Si₃N₄ qui est déposée sur la couche de protection n'a pas pour but de protéger le dispositif contre les attaques chimiques mais seulement de rendre la soudure possible. Ceci est aussi vrai pour l'ensemble des combinaisons de couches décrites dans la table 2. La particularité de Si3N4 est qu'il peut agir à la fois comme couche de protection et comme couche de soudure anodique. Il est cependant difficile de réaliser des dépôts conformes avec du Si3N4, surtout sur des surfaces structurées. Ceux-ci sont pourtant indispensables à l'efficacité protectrice de telles couches. En effet, le moindre défaut peut devenir le point faible du système où vont se concentrer les attaques chimiques. On préfère donc choisir le Ti02 comme matériau de protection dont le dépôt conformal sur une surface structurée est aisé vu qu'il est compatible avec des techniques comme l'ALD.Le Si3N4, qui lui n'est pas compatible avec les méthodes de dépôt conformal de type ALD, peut être déposé en dessus du Ti02, sur la zone de soudure, afin de rendre la soudure anodique possible. (il est connu de la littérature qu'une soudure anodique directement sur de l'oxyde de titane déposée sur Silicium n'est pas réalisable).

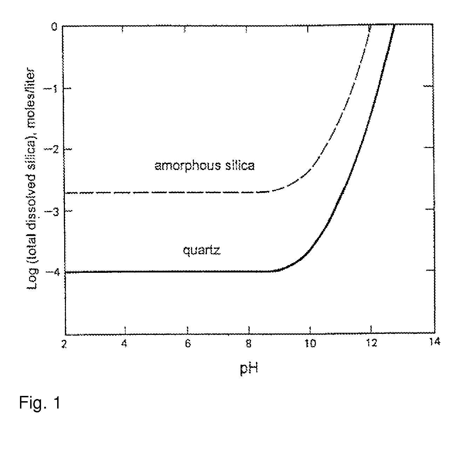

Si le verre est chimiquement inerte par rapport à une solution de pH basique, la solubilité de l'oxyde de silicium, composant essentiel du verre, voit lui sa solubilité augmenter de façon significative avec le pH comme illustré sur la Fig. 1. Par conséquent, la détérioration d'une structure en verre exposée à des solutions de pH basique sur le long terme est un risque qui est adressé dans cette invention. Ainsi, tout comme le silicium, le verre doit être protégé par une couche pouvant résister à une attaque pH basique.

Si le nitrure de silicium ou de l'oxyde de titane peuvent très bien être déposés sur le verre en tant que couche de protection, il est en revanche impossible d'effectuer une soudure anodique directe entre une de plaques protégée et une surface de silicium ayant elle même une couche de protection. Il n'existe aucune couche de protection à notre connaissance applicable sur une plaque de verre qui soit directement compatible avec une soudure anodique. Dans le cas du verre, on utilisera par exemple du nitrure de silicium ou de l'oxyde de titane comme couche de protection que l'on combinera à une fine couche d'oxyde de silicium utilisée comme couche de soudure.

Cette couche de soudure doit permettre d'une part la réalisation de la soudure anodique et d'autre part le maintient de cette soudure au cours du temps malgré son exposition à une solution de pH basique. La couche de soudure doit être suffisamment fine afin de créer des forces capillaires, créant ainsi un stop capillaire de type valve, empêchant une infiltration de la solution basique dans la zone de soudure et évitant par conséquent les risques de délaminage. Comme la soudure anodique induit un changement chimique du matériau présent dans la zone de soudure par la création de liaisons covalentes, le résultat de cette transformation chimique peut changer les propriétés chimico/physique du matériau et le rendre plus résistant aux solutions basiques que sa forme native avant soudure anodique.

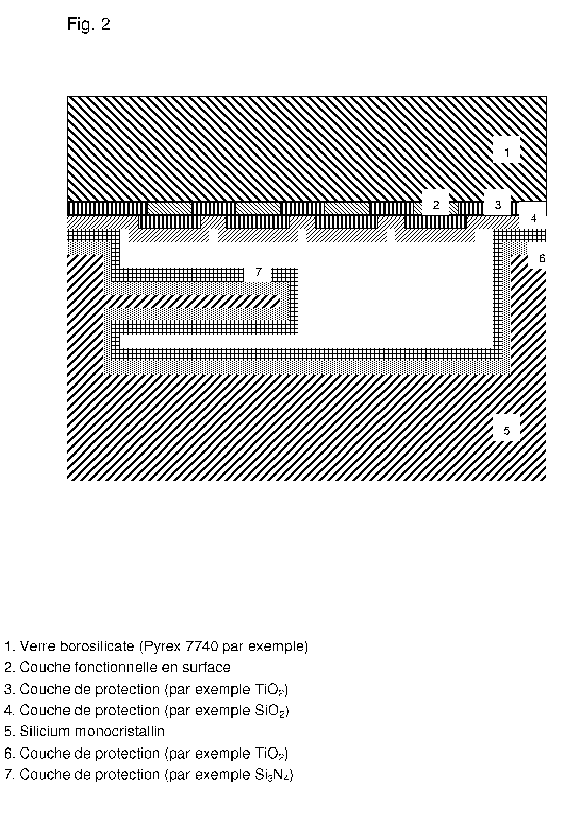

La Fig 2 représente un exemple d'une structure complexe qui peut être protégée et soudée par la technique proposée :
Malgré la présence des couches intermédiaires, il reste possible d'utiliser des paramètres classiques de soudure anodique.
Pour limiter les risques de défaillances locales (pinholes en anglais), on peut utiliser une technique de dépôt conformal appelée ALD (Atomic Layer Deposition) réputée sans pinholes ou lors de l'utilisation d'un dépôt par CVD (Chemical Vapor Deposition) procéder à un dépôt en plusieurs étapes.

### Liste des figures

Fig. 1 Courbe de solubilité du silica et du quartz en fonction du pH
Fig. 2 Vue en coupe d'une structure complexe protégée d'une attaque chimique par des couches minces et scellé par soudure anodique classique.

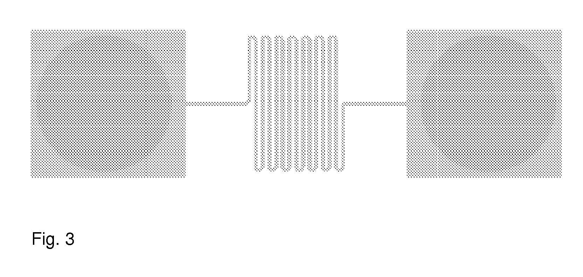

Fig. 3 Véhicule de test utilisé pour mettre en évidence les caractéristiques des couches de protection. Il est constitué de deux entrées fluidiques (les 2 cercles) et d'un canal en forme de serpentin constituant une résistance fluidique.

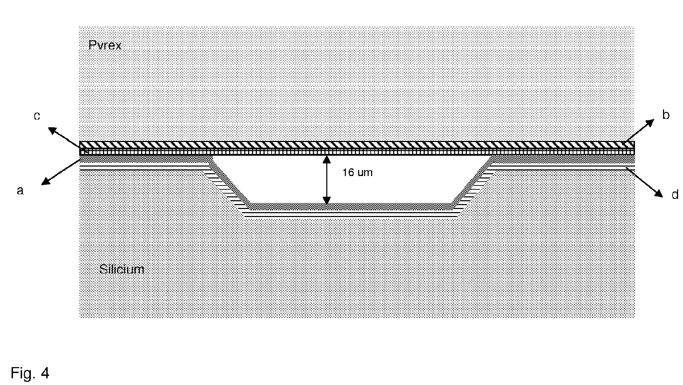

Fig. 4 Schématique de canal constituant la résistance fluidique avec les différentes couches de protection utilisées. Les couches (c) et (b) sont déposées sur le pyrex et les couches (a) et (d) sont déposées sur le Silicium. La couche (a) est préférentiellement du Si3N4 et peut être directement soudée à du Pyrex, ou à la couche (c) qui est la couche de soudure déposée sur la couche de protection (b) qui sert à protéger le Pyrex. La couche (d) est une couche de protection déposée sur le silicium et qu'on peut déposer par ALD mais qui n'est pas directement soudable au Pyrex ou à la couche (c). Par conséquent on peut rajouter sur la couche (d), la couche (a) qui permet cette fois de souder plaque de Silicium ayant une couche de protection non soudable à une plaque de Pyrex protégée ou non.

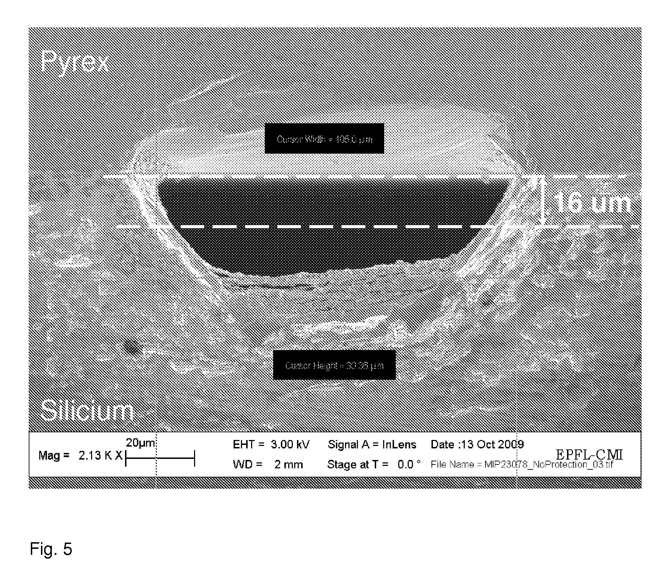

Fig 5, Image SEM de la section du chemin fluidique ayant été exposé à une solution de pH 12 durant 8 jours et ne comportant aucune couche de protection ou de soudure. La résistance fluidique de ce canal ayant diminué de moitié par la gravure du silicium, cette valeur est utilisée comme contrôle par rapport aux autres canaux ayant subit un traitement par couche de protection. La profondeur nominale du canal est de 16 um.

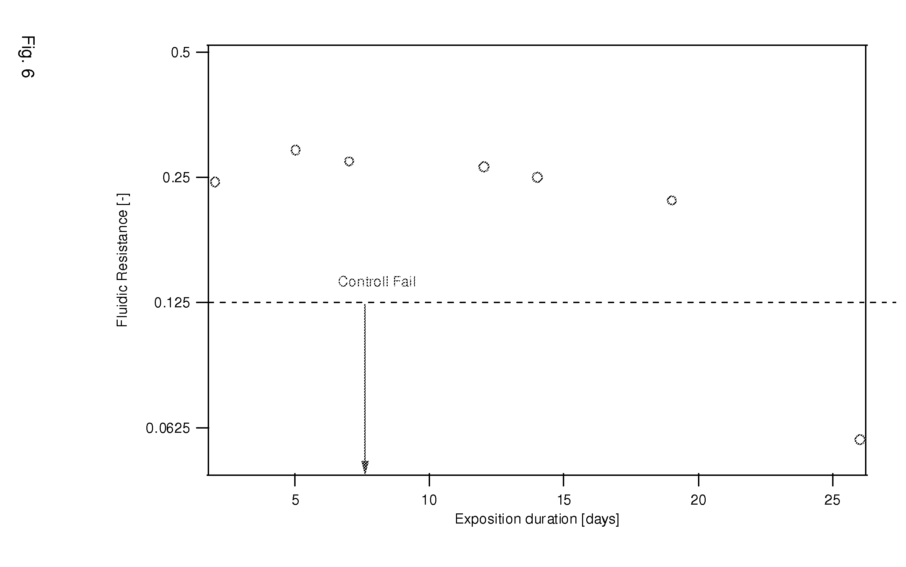

Fig 6 : Evolution de la résistance fluidique en fonction de la durée d'exposition pour un canal avec une couche de protection de 50 nm de Si3N4 déposée sur la plaque de silicium. Le seuil de rupture établi par rapport au contrôle correspond à une diminution de la résistance fluidique d'un facteur 2.

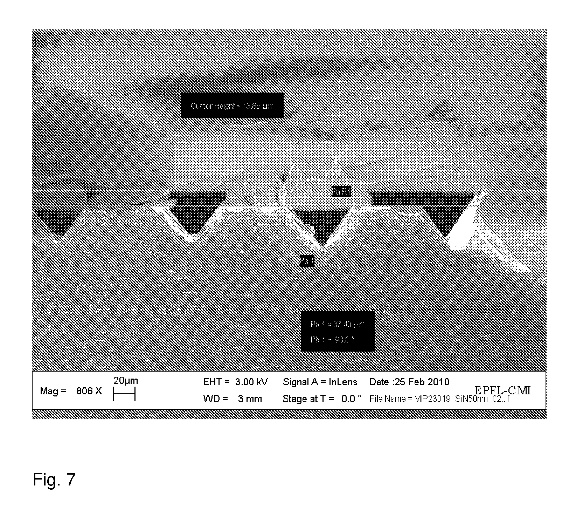

Fig 7 Image SEM de la section du chemin fluidique ayant été exposé à une solution de pH 12 pendant 28 jours et ayant une couche de protection de 50 nm Si3N4 déposée sur le silicium. On observe clairement une attaque anisotrope à l'intersection des canaux constituant le serpentin, alors que le fond des canaux, protégé par le Si3N4, n'est pas attaqué. La défaillance observée suggère qu'une épaisseur de 50 nm ne permet pas d'assurer une bonne protection au niveau de la soudure.

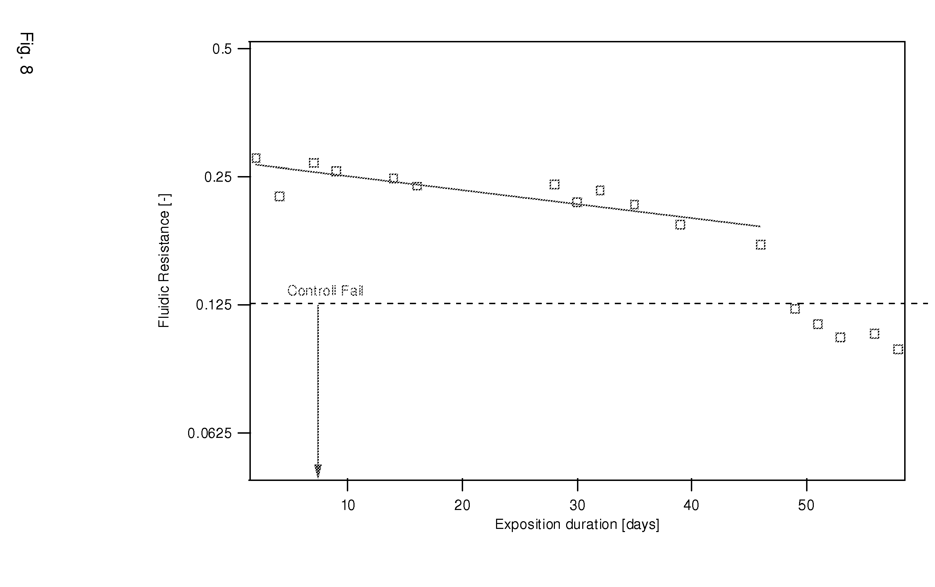

Fig 8 : Evolution de la résistance fluidique en fonction de la durée d'exposition pour un canal avec une couche de protection de 100 nm de Si3N4 déposée sur la plaque de silicium. Le seuil de rupture établi par rapport au contrôle correspond à une diminution de la résistance fluidique d'un facteur 2.

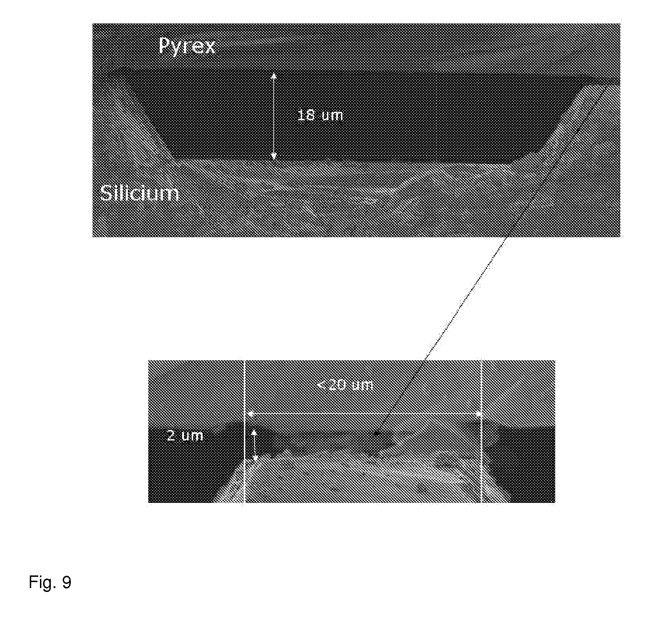

Fig 9 Image SEM de la section du chemin fluidique ayant été exposé à une solution de pH 12 pendant 48 jours et ayant une couche de protection de 100 nm Si3N4 déposée sur le silicium. On observe clairement une attaque du pH à l'interface des plaques définissant les canaux qui constituent le serpentin de résistance fluidique et créant ainsi un court circuit entre deux canaux constituant le serpentin. Par contre on n'observe pas d'attaque anisotrope du Si montrant ainsi que l'épaisseur de 100 nm suffit à assurer une bonne protection de la soudure. Une augmentation de 2 um de la profondeur du canal suggère que le Pyrex est érodé créant ainsi une diminution de la résistance fluidique.

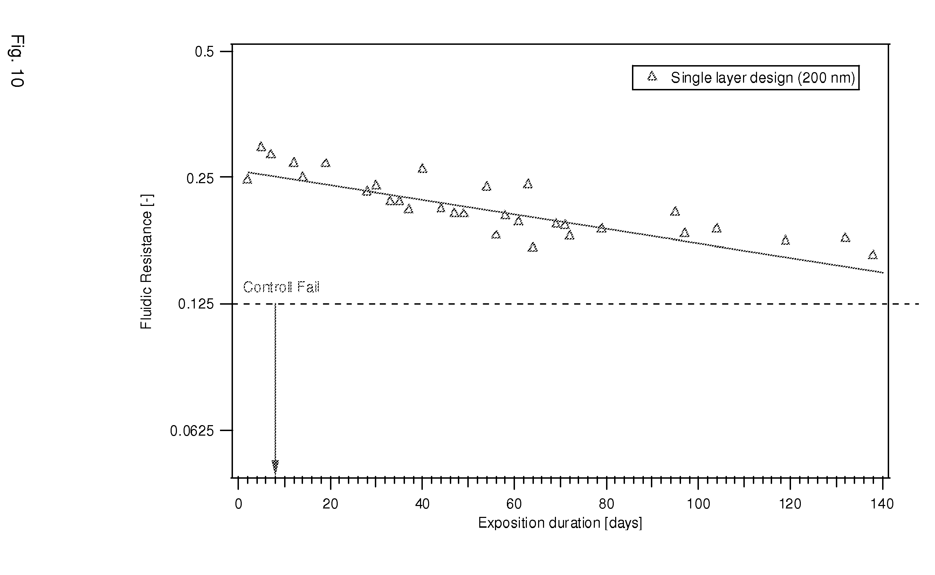

Fig 10 : Evolution de la résistance fluidique en fonction de la durée d'exposition pour un canal avec une couche de protection de 200 nm de Si3N4 déposée sur la plaque de silicium. Le seuil de rupture établi par rapport au contrôle correspond à une diminution de la résistance fluidique d'un facteur 2.

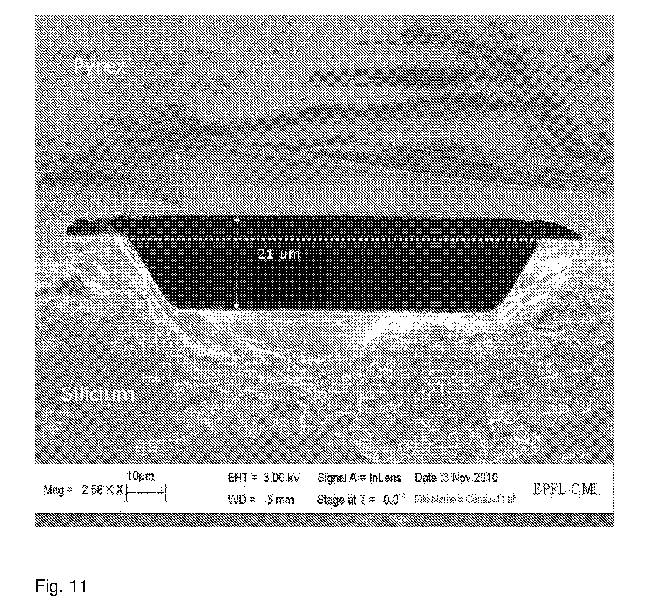

Fig 11 : Image SEM de la section du chemin fluidique ayant été exposé à une solution de pH 12 pendant 140 jours et ayant une couche de protection de 100 nm de Si3N4 déposée sur le silicium. On observe clairement une attaque du pH à l'interface des plaques définissant les canaux qui constituent le serpentin le serpentin de résistance fluidique et une augmentation de plus de 6 um de la profondeur du canal induite par une attaque chimique du Pyrex. La partie protégée du silicium ne semble pas attaquée. Dans ce cas la distance entre les canaux constituant le serpentin est supérieure à 100 um empêchant la création de court-circuit entre les canaux et une chute trop importante de la résistance fluidique comme c'est le cas dans la Fig 9.

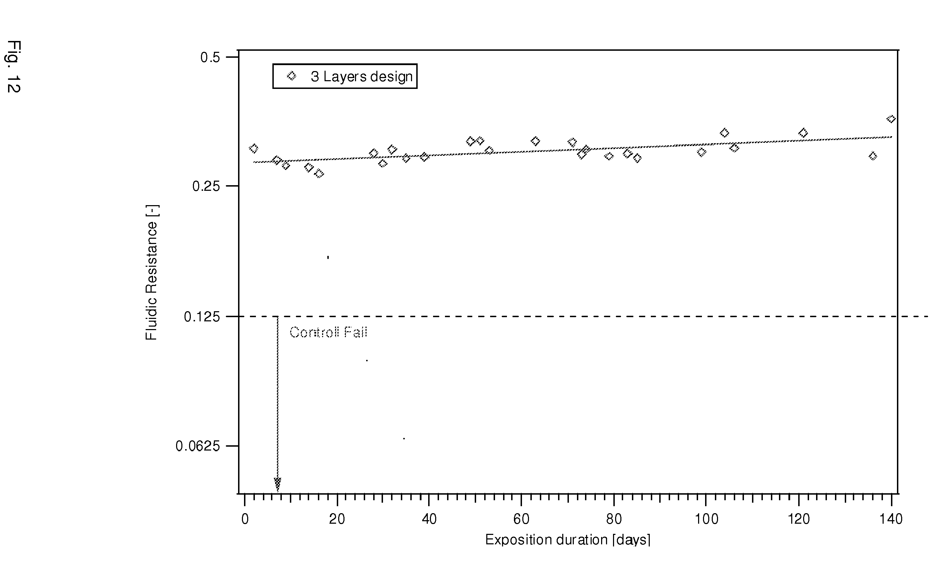

Fig 12 : Evolution de la résistance fluidique en fonction de la durée d'exposition pour un canal avec une couche de protection de 100 nm de Si3N4 déposée sur le silicium, une couche de protection de 200 nm de Ti02 sur le Pyrex et une couche de 100 nm de Si02 déposée sur la couche Ti02 permettant la soudure anodique. Le seuil de rupture établi par rapport au contrôle correspond à une diminution de la résistance fluidique d'un facteur 2.

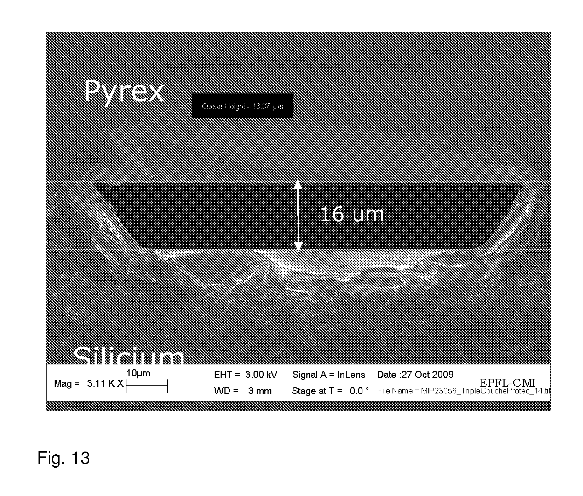

FIG 13 : Image SEM de la section du chemin fluidique ayant été exposé à une solution de pH 12 pendant 140 jours et ayant une couche de protection de 100 nm Si3N4 déposée sur le silicium, une couche de protection de 200 nm de Ti02 sur le Pyrex et une couche de 100 nm de Si02 déposée sur la couche Ti02 permettant la soudure anodique. On voit très bien que le canal a gardé sa profondeur nominale. On montre ainsi que les couches de protection ainsi que la couche de soudure ont rempli parfaitement leur rôle.

### Description détaillée de l'invention

Tout en utilisant une technique de soudure anodique avec des paramètres classiques (350-400°C, 500-1000V) il est possible de souder une plaque silicium contre une plaque de verre (Pyrex 7740) malgré la présence de couches intermédiaires qui servent à la protection contre une attaque chimique.

Le silicium peut être protégé par une couche de TiN fine (< 50nm) ou par toutes sortes de nitrures de silicium (déposés par ALD, PECVD, LPCVD) avec des stoechiométries variables. En utilisant deux couches, on peut aussi le protéger en utilisant un assemblage TiO₂+Si₃N₄ ou TiO₂+a-Si. Avec les épaisseurs allant pour le Ti02 jusqu'à 250nm, jusqu'à 500nm pour la couche supplémentaire de Si₃N₄ (cette épaisseur peut aller jusqu'à <1 µm pour le nitrure de silicium seul) ou <500nm pour la couche supplémentaire de silicium amorphe.

Le Pyrex peut être protégé par deux couches : TiO₂ suivi de SiO₂. Ces deux couches peuvent être déposées par ALD, Sputtering réactif, PECVD (Si02), LPCVD (Si02). La gamme d'épaisseurs utilisable est :
- <500nm pour le SiO₂
- <250nm pour le TiO₂

L'utilisation de l'ALD (Atomic Layer Deposition) comme technique de dépôt est très intéressante dans notre cas puisque le nombre de défauts ponctuels (pinholes en anglais) est réduit drastiquement par rapport aux autres techniques de dépôt mentionnées [R9].

L'utilisation de l'ALD permet aussi d'envisager la protection de structures de formes très complexes puisque la technique est quasi parfaitement conforme (aspect ratio de 1 :1000 démontré [R10]).

Dans le cas où la ou les couches de protection sont déposées par CVD (Chemical Vapor Deposition), il est utile de procéder en plusieurs étapes distinctes. Cela permet, en coupant le vide, de diminuer considérablement les risques de deux défauts (pinholes) superposés.

Il faut aussi noter que cette technique de soudure anodique ne nécessite aucun prétraitement des surfaces pour préparer la soudure. Du moment que les plaques sont exemptes de particules supérieures à 0.5µm il est aisé d'obtenir une soudure fiable et très étanche contrairement à beaucoup d'autres techniques de soudure (Polymer-bonding, Plasma-Activated-Bonding, etc...).

Des tests en pression sur les différentes configurations de soudure n'ont pas montré de différences au niveau de la résistance de la soudure. Il semble donc que quelles que soient les couches protectrices présentes sur le Pyrex ou sur le silicium, la soudure anodique est toute aussi résistante que dans le cas d'une soudure simple sans couche protectrice.

### Résultats Expérimentaux

### I. soudure compatible

Différentes expériences ont mis en évidence la difficulté de souder par soudure anodique du silicium et du verre en y ajoutant des couches intermédiaires. La nature des matériaux, les épaisseurs des couches, l'ordre de dépôt ainsi que les positions relatives des couches par rapport aux plaques sont autant de paramètres clés qu'il faut maitriser pour assurer une soudure anodique fiable. Voilà deux exemples expérimentaux où une soudure tri-couches a pu être réalisée avec succès
- Exemple 1 : Des essais de soudure ont été réalisés sur un ensemble Silicium - Pyrex. Une couche de nitrure de silicium de 100nm a été déposée sur le Silicium. Du coté du Pyrex, une fine couche de Ti02 (50nm) recouvre le substrat. Par-dessus cet ensemble, 100nm de Si02 ont été déposés. Cet ensemble a été soudé à 380°C, à 750V. Les tests au scalpel ont démontré une excellente adhésion.
- Exemple 2 : Une plaque de Silicium a été recouverte d'une couche de 100nm de Ti02 puis d'une couche de 200nm de Si. De même une plaque de Pyrex a été recouverte d'abord d'une couche de 100nm de Ti02 puis d'une autre de 100nm de Si02. Comme précédemment, une soudure à 380°C et 750V a été réalisée.

Les résultats de soudure ont aussi démontré une excellente adhésion entre ces deux plaques.

Plusieurs phénomènes sont cités dans la littérature pour expliquer la soudure anodique. Ci-dessous nous mettons nos résultats expérimentaux en regard avec ces phénomènes.

Premièrement, l'oxydation à l'interface est possible avec l'oxygène venant du Pyrex (en particulier du NaOH dissocié par le champ électrique). Dans notre cas, nous avons constaté que cette théorie permet vraisemblablement d'expliquer certains résultats :
- Le silicium et/ou le Pyrex recouvert de Ti02 ne se soude vraisemblablement pas car le Ti02 empêche l'oxydation à l'interface [R3]
- Le nitrure de silicium doit vraisemblablement empêcher la soudure quand il est déposé sur le Pyrex en empêchant le passage de l'oxygène alors que déposé sur le silicium il est possible de l'oxyder et donc de le souder.
- Cette explication n'est cependant pas totalement satisfaisante dans le cas des soudures multi-couches Si\ TiO2\Si3N4 contre SiO2\ TiO2\Pyrex qui ont été démontrées. En effet si le Ti02 empêche le passage de l'oxygène du Pyrex, comment expliquer que dans cette configuration la soudure ait lieu ? Est-ce que la couche de Si02 déposée par PECVD au-dessus du Ti02 permet de libérer suffisamment de l'oxygène pour permettre à la soudure d'avoir lieu ? Mais alors pourquoi une couche de Si02 plus épaisse empêche la soudure ?

Le deuxième phénomène mis en avant par Veenstra R12 concerne la force électrostatique exercée à l'interface. Lié à l'oxydation des couches à l'interface, la force électrostatique est une clé de compréhension : dans notre cas, le titane déposé sur le Pyrex réduit très fortement la force électrostatique à l'interface.

Un troisième phénomène est la proximité entre les deux plaques. C'est la raison pour laquelle on applique un champ électrique pour obtenir une force électrostatique suffisante à amener en contact étroit les deux plaques à souder. Dans notre cas, la rugosité, qui est un des aspects de la proximité, pourrait avoir de l'impact : la différence de rugosité entre des dépôts ALD et par pulvérisation est connue, mais ne semble pas jouer un rôle important dans notre cas.

### II. Résistance fluidique

Afin de mettre en évidence la qualité de la protection et de la soudure à pH élevé un véhicule de test constituant une résistance fluidique a été utilisé (Fig 3). Ce dernier a permis de mettre en évidence les défaillances et les performances des différentes configurations utilisées.

Le véhicule de test a été soumis à une solution de pH 12 qui représente une forme accélérée d'étude par rapport à un pH moins basique dans l'attaque chimique du Silicium. De plus, en ce qui concerne verre, on voit selon la Fig 1, qu'à partir du pH 9, la solubilité du dioxyde de silicium augmente exponentiellement. Par conséquent les résultats obtenus à pH 12 représentent un facteur d'accélération d'au moins 1000 par rapport à un pH de 9 pouvant correspondre à une solution plus représentative d'un système d'injection de médicament.

Le canal en question peut comporter 4 différentes couches (a), (b), (c) et (d) comme représenté dans la Fig 4. Comme aucune couche de protection (b) appliquée sur le pyrex ne peut être directement soudée sur la plaque de Silicium (et ceci quelque soit la configuration (a) - (d)), la couche (b) doit être automatiquement recouverte par une couche de soudure (c). Dans le cas du silicium, la couche de protection (a) peut être directement soudée au Pyrex sans protection ou à la couche de soudure (c). Dans le cas ou le silicium est couvert par une couche (d) ne pouvant pas être soudé, la couche (a) peut être déposé sur la couche (d) et être utilisée comme couche de soudure.

La Fig 5 montre l'attaque à pH 12 d'un canal n'ayant aucune protection. La résistance fluidique de ce canal ayant diminué d'un facteur 2, il est utilisé comme contrôle pour déterminer le seuil de défaillance pour les designs de canaux comportant des couches de protection.

La Fig 6 montre qu'un design comprenant uniquement une couche de protection Si3N4 (a) de 50 nm atteint le seuil de défaillance après 22 jours. Comme illustré dans la Fig 7, la défaillance est provoquée par une gravure anisotrope entre les canaux, montrant ainsi que la faiblesse se situe au niveau de la soudure. Par contre la couche de protection au fond du canal ne semble pas avoir été attaquée par rapport au contrôle de la Fig 5.

La Fig 8 montre qu'un design comprenant uniquement une couche de protection Si3N4 (a) de 100 nm atteint le seuil de défaillance après 48 jours. Comme illustré dans la Fig 9, la défaillance est provoquée par la création d'un court-circuit entre les canaux. Le canal, dont la profondeur a augmenté de 2 microns, semble avoir subit cette attaque du côté de la plaque en Pyrex, tandis que le côté de la plaque en Silicium protégée semble intact. Ce résultat suggère qu'une épaisseur de 100 nm suffit à assurer une bonne tenue de la couche de protection au niveau de la soudure du côté de la plaque en Silicium contrairement la soudure précédemment testée avec de 50 nm de Si3N4. La défaillance résulte probablement d'une attaque de la plaque de Pyrex non protégée.

La Fig 13 montre qu'un design comprenant une couche de protection Si3N4 (a) de 100 nm sur le silicium, une couche de protection Ti02 de 200 nm (b) et une couche de soudure Si02 50 nm (c) sur la plaque de Pyrex maintient une résistance fluidique supérieur ou égale à sa valeur nominale au cours du temps lorsqu'il exposé à une solution de pH 12. En effet sur plus de 140 jours, la résistance fluidique du serpentin ne diminue pas, contrairement aux designs précédemment expérimentés. La légère augmentation de la résistance fluidique est plutôt attribuée à des artefacts du set-up comprenant un filtre en amont du chip qui peut, avec le temps, partiellement se bloquer pour donner la tendance observée sur la Fig 12.

Comme illustré dans la Fig 14, le canal formant le serpentin, conserve, après plus de 140 jours d'incubation à pH de 12, ses dimensions nominales suggérant ainsi que l'ensemble des couches de protection, ainsi que celle de soudure ont parfaitement rempli leur fonction.

### Références

R1. S.Weichel, R. de Reus, S.Bouaidat, P.A.Rasmussen, O.Hansen, K.Birkelund, H.Dirac, Low-temperature anodic bonding to silicon nitride, in : Sensors and Actuators A, 82 (2000) 249-253
R2. A.Berthold, L.Nicola, P.M.Sarro, M.J.Vellekoop, Glass-to-glass anodic bonding with standard IC technology thin films as intermediate layers, in: Sensors and Actuators A, 82 (2000) 224-228
R3. K.M.Knowles, A.T.J. van Helvoort, Anodic bonding, in: International Materials Reviews, 51-5 (2006) 273-310
R4. T.N.H.Lee, D.H.Y.Lee, C.Y.N.Liaw, A.I.K.Lao, I.M.Hsing, Detailed characterization of anodic bonding process between glass and thin-film coated silicon substrates, in: Sensors and Actuators A, 86 (2000) 103-107
R5. D.J.Lee, Y.H.Lee, J.Jang, B.K.Ju, Glass-to-glass electrostatic bonding with intermediate amorphous silicon film for vacuum packaging of microelectronics and its application, in: Sensors and Actuators A, 89 (2001) 43-48
R6. R.Legtenberg, S.Bouwstra, M.Elwenspoek, Low-temperature glass bonding for sensors applications using boron oxide thin films, in: J. Micromech. Microeng., 1 (1991) 157-160
R7. A.Hanneborg, M.Nese, H.Jakobsen, R.Holm, Review: Silicon-to-thin film anodic bonding, in: J. Micromech. Microeng., 2 (1992) 117-121
R8. M.Wiegand, M.Reiche, U.Gösele, K.Gutjahr, D.Stolze, R.Longwitz, E.Hiller, Wafer bonding of silicon wafers covered with various surface layers, in: Sensors and Actuators A, 86 (2000) 91-95
R9. X.Du, K.Zhang, K.Holland, T.Tombler, M.Moskovits, Chemical corrosion protection of optical components using atomic layer deposition, in: Applied Optics, 48-33 (2009) 6470-6477
R10. J.W.Elam, D.Routkevitch, P.P.Mardilovich, S.M.George, Conformal coating on ultrahigh-aspect-ratio nanopores of anodic alumina by atomic layer deposition, in: Chem. Mater., 15 (2003) 3507-3517
R11. T.Bork, F.Bianchi, Fluidic capillary chip for regulating drug flow rates of infusion pumps, European Patent EP2138198
R12. T.T.Veenstra & al., "Use of selective anodic bonding to create micropump chambers with virtually no dead volume", J. Electrochem. Soc., 2000, 148 (2) p.G68-G72

## Revendications

1. Dispositif comprenant une plaque comprenant une surface de silicium et une plaque comprenant une surface de verre fixées l'une à l'autre, la zone de fixation ainsi formée entre les plaques définissant une structure multicouche comprenant une première couche de protection contre une altération physique du matériau couvrant la surface de silicium et une deuxième couche de protection contre une altération physique du matériau couvrant la surface de verre; ladite structure multicouche comprenant en outre au minimum une couche additionnelle permettant de réaliser une soudure anodique entre les deux couches de protection.

2. Dispositif selon la **revendication 1**, de type MEMS, dans lequel les plaques sont usinables.

3. Dispositif selon les **revendications précédentes** dans lequel le matériau constituant les couches de protection qui couvre la surface de verre et silicium résiste à des pH acides et/ou basiques

4. Dispositif selon les **revendications précédentes** dans lequel ledit matériau constituant les couches de protection comprend par exemple du dioxyde de titane, du nitrure de titane ou du nitrure de silicium

5. Dispositif selon les **revendications précédentes** dans lequel ladite couche de soudure est déposée uniquement sur la couche protection recouvrant la plaque de verre

6. Dispositif selon les **revendications précédentes** dont la couche de soudure ne résiste pas à un pH basique.

7. Dispositif selon les **revendications 1 à 6** dont la couche de soudure a une épaisseur permettant de créer en cas d'attaque de cette couche un stop capillaire au niveau de la soudure.

8. Dispositif selon les **revendications 1 à 6** dont la couche de soudure est composé d'un matériau subissant une transformation chimique au niveau de la soudure durant la soudure anodique en le rendant résistant aux solutions basiques.

9. Dispositif selon les **revendications précédentes** dont la couche de soudure est du dioxyde de silicium.

10. Dispositif selon les **revendications 1 à 4 et 9** dans lequel ladite couche de soudure est déposée uniquement sur la couche de protection recouvrant la plaque de silicium

11. Dispositif selon les **revendications 1 à 4 et 9 à 10** dans lequel ladite couche de soudure est également une couche de protection.

12. Dispositif selon les **revendications 1 à 4 et 9 à 11** dont la couche de soudure est du silicium nitrure de Silicium

13. Dispositif selon les **revendications 1 à 4 et 9 à 12** dans lequel la couche soudure a une épaisseur supérieure ou égale à 100 nm lorsqu'elle est appliquée directement sur la surface Silicium.

14. Dispositif selon **les revendications précédentes** dans lequel la plaque avec surface de verre est en borosilicate tel que Pyrex ou en silicium.

15. Dispositif selon **les revendications précédentes** dans lequel la plaque avec surface de Silicium est en Silicium sur isolant ou en verre.

16. Dispositif selon l'une des **revendications précédentes** dans lequel ladite structure multicouche a une épaisseur inférieure à 1µm.

17. Dispositif selon l'une des **revendications précédentes** dans lequel les couches de protections et de soudures sont biocompatibles

18. Dispositif selon les **revendications précédentes** utilisé comme système médical.

19. Dispositif selon la **revendication précédente** utilisé comme système médical implantable.

20. Procédé de fabrication d'un dispositif type MEMS comprenant les étapes suivantes :
- appliquer une couche de protection contre une attaque chimique sur au moins une région en silicium d'une face principale d'une première plaque,
- appliquer une couche de protection contre une attaque chimique sur au moins une région en verre d'une face principale d'une deuxième plaque,
- ajouter un matériau permettant de réaliser une soudure anodique entre les deux couches de protection.

21. Procédé selon la **revendication 20** dans lequel les deux couches de protection, ainsi que la couche supplémentaire de soudure, sont appliquées par l'une ou la combinaison des techniques suivantes : Low Pressure Chemical Vapour Deposition (LPCVD), Plasma Enhanced Chemical Vapour Deposition (PECVD), Atomic Layer Deposition (ALD), oxidation, evaporation or sputtering.
**Table 1 Des exemples de verres disponibles sur le marché et de la céramique de verre utilisée pour la liaison anodique**
| **Manufacturer** | **Code** |
|---|---|
| Corning | No. 0080 |
| | No. 0120 |
| | No. 1720 |
| | No. 1729 |
| | No. 1737 |
| | No. 7052 |
| | No. 7056 |
| | No. 7059 |
| | No. 7070 |
| | No. 7740 (Pyrex) |
| | |
| | No. 9626 |
| | No. 9741 |
| | Zerodur |
| | glass ceramic |
| Hoya | SD-2 |
| Matsunami | No. 0700 |
| Schott | Borofloat |
| | Forturan |
| | No. 8329 |
| | No. 8330 (Tempax) |
**Table 2 Exemples de divers matériaux utilisés comme couche intermédiaire en liaison anodique**
| **Bond type** | **Interlayer material** |
|---|---|
| Si-glass | Al |
| | Al/Ti |
| | Hf |
| | Mg |
| | Schott no. 8329 glass |
| | Si (amorphous) |
| | Si (polycrystalline) |
| | SiC (sputtered) |
| | Si₃N₄ |
| | SiNₓ |
| | SiO₂ (dry and wet thermally grown) |
| | SiO₂ (sputtered) |
| | Sn |
| Si-Si | Iwaki no. 7570 glass (sputtered) |
| | Lithium glass (sputtered) |
| | Pyrex-like-glasses (sputtered or evaporated) |
| | Pyrex-like-glasses with Al, Poly-Si, Si₃N₄, SiO₂ |
| | SiO₂ (dry and wet thermally grown) |
| | SiO₂ (sputtered) |
| Glass-glass | Al |
| | ITO/a-Si |
| | Ni-Cr |
| | Poly-Si, Si₃N₄, SiC, a-Si |
